# EUROPÄISCHE PATENTSCHRIFT

(11) **EP 0 945 416 B1**
(45) Veröffentlichungstag und Bekanntmachung des Hinweises auf die Patenterteilung: **12.06.2002**
(21) Anmeldenummer: 99103752.4
(22) Anmeldetag: 26.02.1999
(51) Int. Cl.: C07C 17/12, C07C 25/13

(54) **Verfahren zur Herstellung von 2,4-Dichlor-3,5-dimethylfluorbenzol**
Process for the preparation of 2,4-dichloro-3,5-dimethylfluorobenzene
Procédé pour la préparation de 2,4-dichloro-3,5-diméthylfluorobenzène

(30) Priorität: 11.03.1998 DE 19810392
(43) Veröffentlichungstag der Anmeldung: 29.09.1999
(73) Patentinhaber: Bayer Aktiengesellschaft, 51368 Leverkusen (DE)
(72) Erfinder: Mais, Franz-Josef Dr., 40591 Düsseldorf (DE); Bloodworth, Robert Horst Dr., 51061 Köln (DE); Von dem Bruch, Karsten Dr., 51375 Leverkusen (DE)

(56) Entgegenhaltungen:
- EP-A- 0 340 581
- EP-A- 0 657 407
- EP-A- 0 718 261
- WO-A-98/47862
- DATABASE WPI Section Ch, Week 8533 Derwent Publications Ltd., London, GB; Class E13, AN 85-199994 XP002104628 & JP 60 125251 A (IHARA CHEM IND CO LTD) , 4. Juli 1985

## Beschreibung

Die vorliegende Erfindung betrifft ein Verfahren zur Herstellung von 2,4-Dichlor-3,5-dimethylfluorbenzol durch selektive Chlorierung von 3,5-Dimethylfluorbenzol in Gegenwart bestimmter Katalysatoren.

2,4-Dichlor-3,5-dimethylfluorbenzol ist ein Zwischenprodukt für die Herstellung von pharmazeutischen Wirkstoffen des Chinoloncarbonsäuretyps (siehe die eigene ältere deutsche Patentanmeldung Nr. 197 17 231.8).

Die Herstellung von 2,4-Dichlor-3,5-dimethylfluorbenzol ist aus vorveröffentlicher Literatur nicht bekannt. Die vorgenannte eigene ältere Patentanmeldung beschreibt die Herstellung von 2,4-Dichlor-3,5-dimethyl-fluorbenzol ohne den Einsatz von Co-Katalysatoren.

Die selektive Herstellung der ähnlichen Verbindung 3-Fluor-2,4-dichlortoluol mit reduzierter Bildung von 3-Fluor-2,6-dichlortoluol ist aus EP-A 657 407 und in einem verbesserten Verfahren aus EP-A 718 261 bekannt.

Der Nachteil dieser Verfahren ist jedoch, dass die Chlorierung diskontinuierlich in zwei verschiedenen Temperaturbereichen durchgeführt werden muß.

Es bestand daher das Bedürfnis ein selektives Verfahren für die Herstellung von 2,4-Dichlor-3,5-dimethylfluorbenzol aus 3,5-Dimethyl-fluorbenzol zu entwickeln.

Es wurde nun ein Verfahren zur Herstellung von 2,4-Dichlor-3,5-dimethylfluorbenzol gefunden, das dadurch gekennzeichnet ist, daß man 3,5-Dimethylfluorbenzol in Gegenwart von Friedel-Crafts-Katalysatoren und einem schwefelhaltigen Co-Katalysator chloriert.

3,5-Dimethylfluorbenzol ist im Handel erhältlich.

Friedel-Crafts-Katalysatoren für das erfindungsgemäße Verfahren sind als solche bekannt. Beispiele sind: Antimonchloride, Antimonoxychloride, Aluminiumchlorid, Eisen(II)-chlorid, Eisen(III)-chlorid, Tellurchloride, Bleichloride, Molybdänchloride, Zinnchloride, Wolframchloride, Titanchloride, Zinkchloride, Bortrichlorid und Bortrifluorid. Es können auch Elemente und Elementverbindungen, die während der Chlorierung einen Friedel-Crafts-Katalysator (= Lewis-Säure) bilden, eingesetzt werden (Vorläufer für Friedel-Crafts-Katalysatoren), beispielsweise die Metalle oder Halbmetalle Antimon, Eisen, Blei, Zinn, Zink, Molybdän, Tellur oder Aluminium oder deren Oxide, Sulfide, Carbonyle oder Salze (beispielsweise Carbonate).

Beispiele für in Frage kommende Elementarverbindungen sind: Antimonoxide, Eisenoxide, Eisensulfide, Bleisulfide, Zinnsulfide, Zinksulfide, Eisencarbonyle, Molybdäncarbonyle und Borphosphat. Anstelle der erwähnten Chloride können auch die entsprechenden Fluoride, Bromide und gegebenenfalls lodide der genannten Elemente eingesetzt werden. Bevorzugt werden in das erfindungsgemäße Verfahren Antimonchloride, Eisen, Eisenoxide, Eisensulfide, Eisencarbonyle und Eisen(III)-chlorid eingesetzt. Besonders bevorzugt ist Eisen(III)-chlorid. Friedel-Crafts-Katalysatoren und/oder Vorläufer davon können einzeln oder als beliebige Gemische untereinander eingesetzt werden.

Die Menge des Friedel-Crafts-Katalysators bzw. seines Vorläufers kann in weiten Grenzen variiert werden. So ist häufig bereits bei einem Zusatz von 0,0005 Gew.-% eine Katalysatorwirkung erkennbar. Andererseits können auch 5 Gew.-% oder mehr des Friedel-Crafts-Katalysator zugesetzt werden, jedoch bieten solche hohen Mengen im allgemeinen keinen Vorteil, bringen aber gegebenenfalls bei der Aufarbeitung Nachteile mit sich. Üblicherweise wird der Friedel-Crafts-Katalysator in einer Menge von 0,001 bis 1,0 Gew.-%, bevorzugt 0,01 bis 0,5 Gew.-% eingesetzt. Alle diese Mengenangaben sind auf die Menge des eingesetzten 3,5-Dimethylfluorbenzols bezogen.

Als schwefelhaltige Co-Katalysatoren für das erfindungsgemäße Verfahren können elementarer Schwefel in seinen verschiedenen Formen oder Schwefelhalogenide, z.B. Schwefeldichlorid, Schwefeltetrachlorid, Dischwefeldichlorid oder Dischwefeldibromid, eingesetzt werden.

Als Co-Katalysatoren können weiterhin z.B. Heterocyclen eingesetzt werden, die gleichzeitig N- und S-Atome enthalten und z.B. den Klassen der Thiazine, Thiazepine, Thiazocine, Phenoxythiine oder Thianthrene angehören. Beispielsweise seien genannt:

Thiazepine der Formeln 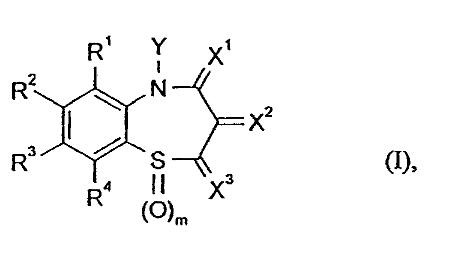 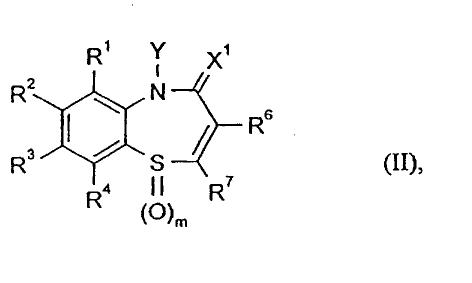 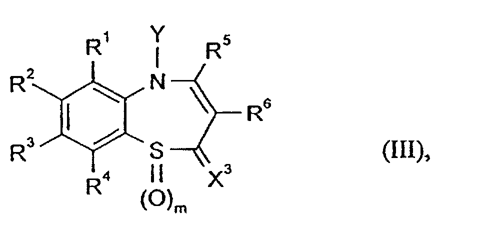 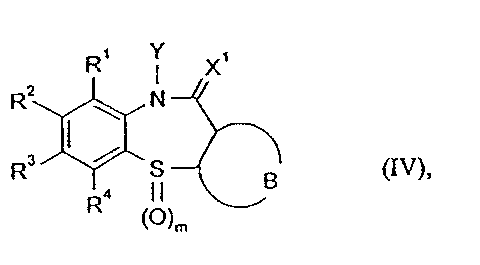 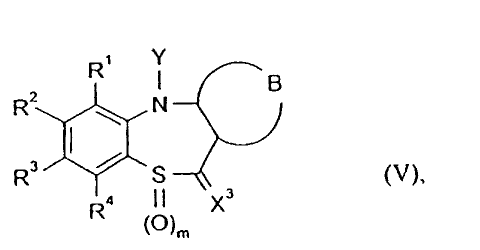 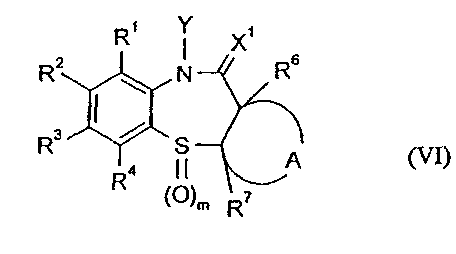 und 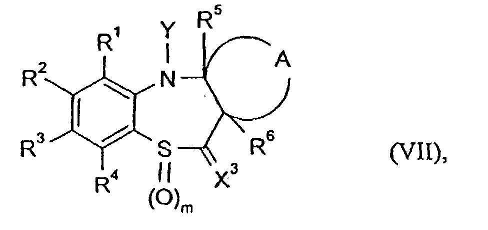 worin
- R¹, R², R³, R⁴: gleich oder verschieden sind und für Wasserstoff, Hydroxy, Amino, Cyano, Halogen, Nitro, Nitroso, Sulfonyl, Sulfoxyl, Tosyl, Mercapto, Carboxyl, Carboxyamid, Carbalkoxy, Dithiocarboxyl, Thiocarboxyamid, Dithiocarbalkoxy, gegebenenfalls substituiertes Alkyl, Aryl, Heteroaryl, Alkoxy, Aryloxy, Heteroaryloxy, Acyloxy, Alkylthio, Arylthio, Heteroarylthio, Acylthio, Acyl, Thioacyl oder Acylamino stehen und weiterhin untereinander einen oder mehrere gesättigte oder ungesättigte, gegebenenfalls substituierte isocyclische oder heterocyclische Kohlenstoffringe mit bis zu 8 C-Atomen bilden können,
- Y: Wasserstoff, gegebenenfalls substituiertes Alkyl, Aryl, Heteroaryl, Acyl, Thioacyl, Acyloxy, Arylamino oder Acylamino bedeutet,
- X¹, X² und X³: unabhängig voneinander jeweils eine der folgenden Gruppierungen bedeutet: 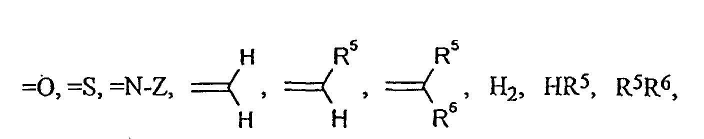
- R⁵, R⁶ und R⁷: gleich oder verschieden sind und die Bedeutung von R¹ bis R⁴ besitzen, mit der Ausnahme, daß sie untereinander keine Ringe bilden können,
- Z: die Bedeutung von Y hat mit der Ausnahme, daß Z nicht gleich H sein kann,
- A: die Anellierung eines gegebenenfalls substituierten gesättigten isocyclischen oder heterocyclischen Rings mit bis zu 8 C-Atomen bedeutet,
- B: die Anellierung eines gegebenenfalls substituierten ungesättigten isocyclischen oder heterocyclischen Rings mit bis zu 8 C-Atomen bedeutet und
- m: Null oder 1 bedeutet.

Cyclische benzokondensierte Imine der Formel 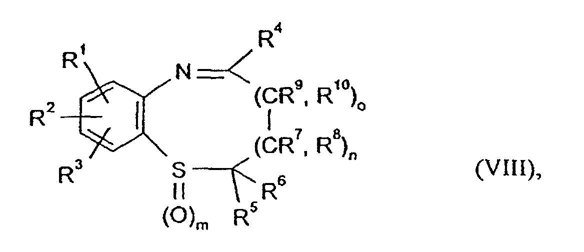 in der
- R¹ und R²: unabhängig voneinander Wasserstoff, Hydroxy, Amino, Cyano, Halogen, Nitro, Carboxyl, Halogenocarbonyl, Carboxyamid, Alkoxycarbonyl, Alkyl, Aryl, Alkoxy, Aryloxy, Acyloxy, Alkylthio, Arylthio, Acylthio, Acyl, Thioacyl oder Acylamino bedeuten,
- R³: für Wasserstoff oder Chlor steht und weiterhin mit einem der Reste R¹ oder R² bei benachbarter Substitution und gemeinsam mit den substituierten C-Atomen einen anellierten gesättigten, ungesättigten oder aromatischen isocyclischen oder heterocyclischen Ring mit 5 bis 8 Ringatomen bilden kann,
- R⁴: Wasserstoff, Alkyl, Aryl, Halogen, Alkylthio, Arylthio, Alkoxy, Aryloxy, Amino, Hydrazino, Alkylhydrazino oder Phenylhydrazino bedeutet,
- m, n und o: unabhängig voneinander den Wert Null oder 1 annehmen können,
- R⁵, R⁷ und R⁹: unabhängig voneinander Wasserstoff, Alkyl, Alkoxy, Phenyl, Acyloxy, Cyano, Halogen, Carboxyl, Alkoxycarbonyl, Phenoxy oder Acyl bedeuten, wobei R⁵ und R⁷ oder R⁷ und R⁹ gemeinsam mit den substituierten C-Atomen einen gesättigten, ungesättigten oder aromatischen isocyclischen oder heterocyclischen Ring mit 5 bis 8 Ringatomen bilden können,
- R⁶, R⁸ und R¹⁰: unabhängig voneinander Wasserstoff, Alkyl oder Halogen bedeuten, wobei R⁶ und R⁸ oder R⁸ und R¹⁰ gemeinsam eine Doppelbindung bilden können und wobei weiterhin R⁵ und R⁶ gemeinsam doppelt gebundenen Sauerstoff, Schwefel oder R¹¹-subsituierten Stickstoff darstellen können, wobei R¹¹ Alkyl, Aryl, Acyl, Alkylamino oder Arylamino bedeutet.

Benzo[f]-1,4-thiazepine der Formel 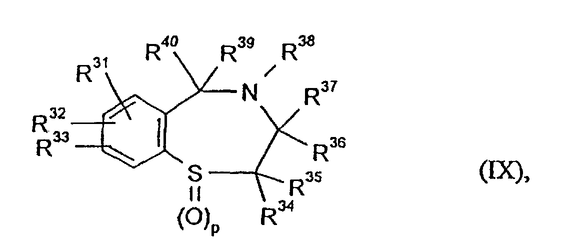 in der
- R³¹ und R³²: unabhängig voneinander Wasserstoff, Hydroxy, Amino, Cyano, Halogen, Nitro, C₁-C₈-Alkyl, nicht substituiertes oder durch R³¹ und R³² substituiertes Phenyl (mit Ausnahme der erneuten Substitution durch R³¹- und R³²-substituiertes Phenyl), C₁-C₈-Alkoxy, Phenoxy, C₁-C₈-Acyloxy, C₁-C₈-Acyl oder C₁-C₈-Alkoxycarbonyl bedeuten,
- R³³: für Wasserstoff oder Chlor steht und weiterhin mit einem der Reste R³¹ oder R³² und gemeinsam mit den substituierten C-Atomen einen anellierten gesättigten, ungesättigten oder aromatischen isocyclischen oder heterocyclischen Ring mit 5 bis 8 Ringatomen bilden kann,
- R³⁴, R³⁶ und R⁴⁰: unabhängig voneinander Wasserstoff, C₁-C₈-Alkyl, nicht substituiertes oder durch R³¹ und R³² substituiertes Phenyl (mit Ausnahme der erneuten Substitution durch R³¹- und R³²-substituiertes Phenyl), C₁-C₈-Acyl, C₁-C₈-Alkoxycarbonyl, Cyano, Halogen, Carboxyl, C₁-C₈-Alkoxy, C₁-C₈-Alkylthio, Phenylthio, Benzylthio, Phenoxy oder C₁-C₈-Acyloxy bedeuten,
- R³⁵, R³⁷ und R³⁹: unabhängig voneinander Wasserstoff, C₁-C₈-Alkyl, Halogen, C₁-C₈-Alkoxy oder C₁-C₈-Alkylthio bedeuten.
- R³⁸: Wasserstoff, C₁-C₈-Alkyl, nicht substituierte oder durch R³¹- und R³²-substituiertes Phenyl (mit Ausnahme der erneuten Substitution durch R³¹- und R³²-substituiertes Phenyl), C₁-C₈-Acyl, C₁-C₈-Thioacyl, Halogencarbonyl oder C₁-C₈-Alkoxycarbonyl bedeutet und
- p: für eine der Zahlen Null oder 1 steht,
wobei weiterhin
die Substituentenpaare R³⁴ und R35, R³⁶ und R³⁷ sowie R³⁹ und R⁴⁰ unabhängig voneinander doppelt gebundenen Sauerstoff, Schwefel oder R³⁸-substituierten Stickstoff bedeuten können und wobei weiterhin
die Substituentenpaare R³⁵ und R³⁶ sowie R³⁸ und R³⁹ unabhängig voneinander eine Doppelbindung bilden können und wobei weiterhin
die Substituentenpaare R³⁴ und R³⁷ sowie R³⁸ und R³⁹ unabhängig voneinander 3-bis 5-gliedriges Alkylen bilden können, bei dem 1 oder 2 C-Atome durch Sauerstoff, Schwefel oder R³⁸-substituierten Stickstoff ersetzt sein können, und wobei weiterhin

R⁴⁰ auch die Bedeutung Hydrazino, C₁-C₈-Alkylhydrazino oder Phenyl-hydrazino annehmen kann.

Am exocyclischen N-Atom oxysubstituierte cyclische Amidine der Formel 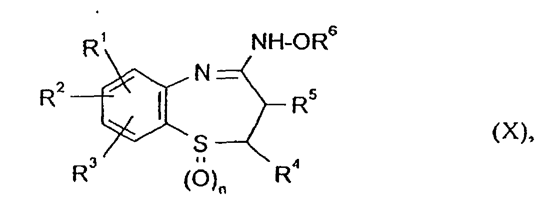 in der
- R¹ und R²: unabhängig voneinander Wasserstoff, Cyano, Halogen, Carboxyl, Alkoxycarbonyl, Alkyl, Aryl, Alkoxy, Aryloxy oder Acyl bedeuten,
- R³: für Wasserstoff, Alkyl oder Chlor steht und weiterhin mit einem der Reste R¹ oder R² bei benachbarter Substitution und gemeinsam mit den substituierten C-Atomen einen anellierten gesättigten, ungesättigten oder aromatischen, isocyclischen oder heterocyclischen Ring mit 5 bis 8 Ringatomen bilden kann,
- R⁴ und R⁵: unabhängig voneinander Wasserstoff, Alkyl, Aryl, Halogen, Alkoxy, Aryloxy, Acyl oder Acyloxy bedeuten oder gemeinsam mit den substituierten C-Atomen einen gesättigten oder ungesättigten, isocyclischen oder heterocyclischen Ring mit 5 bis 8 Ringatomen bilden können,
- R⁶: Wasserstoff, Alkyl, Aryl oder durch Alkyl oder Aryl substituiertes Silyl bedeutet und
- n: den Wert Null oder 1 annehmen kann.

1,6-Benzo-thiazocine der Formel 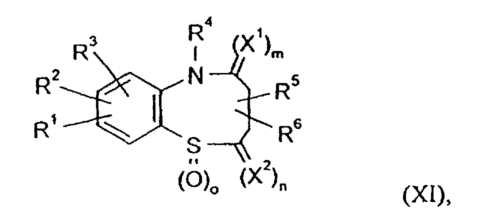 in der
- R ¹ und R²: unabhängig voneinander Wasserstoff, Hydroxy, Amino, Cyano, Halogen, Nitro, Alkylsulfonyl, Phenylsulfonyl, Alkylsulfoxyl, Phenylsulfoxyl, Tosyl, Mercapto, Carboxyl, Halogencarbonyl, Carboxyamid, Alkoxycarbonyl, Thiocarboxyamid, Alkyl, Aryl, Heteroaryl, Alkoxy, Aryloxy, Heteroaryloxy, Acyloxy, Alkylthio, Arylthio, Heteroarylthio, Acylthio, Acyl, Thioacyl oder Acylamino bedeuten,
- R³: für Wasserstoff oder Chlor steht und weiterhin mit einem der Reste R¹ oder R² und gemeinsam mit den substituierten C-Atomen einen anellierten gesättigten, ungesättigten oder aromatischen isocyclischen oder heterocyclischen Ring mit 5 bis 8 Ringatomen bilden kann,
- R⁴: Wasserstoff, Alkyl, Aryl, Heteroaryl, Acyl, Thioacyl, Halogencarbonyl oder Alkoxycarbonyl bedeutet,
- X¹ und X²: unabhängig voneinander für doppelt gebundenen Sauerstoff, Schwefel oder R⁷-substituierten Stickstoff stehen wobei R⁷ den Bedeutungsumfang von R⁴ mit Ausnahme von Wasserstoff hat,
- m, n und o: unabhängig voneinander jeweils den Wert Null oder 1 annehmen können und
- R⁵ und R⁶: unabhängig voneinander an einem oder an zwei der zwischen dem S- und dem N-Atom im 8-Ring befindlichen C-Atome stehen können, sofern diese C-Atome nicht durch X¹ bzw. X² besetzt sind, und den Bedeutungsumfang von R¹ bzw. R² haben, wobei bei benachbarter Substitution auch mit den substituierten C-Atomen ein gesättigter, ungesättigter oder aromatischer isocyclischer oder heterocyclischer Ring mit 5 bis 8 Ringatomen gebildet werden kann und wobei weiterhin R⁵ und R⁶ gemeinsam auch doppelt gebundenen Sauerstoff oder Schwefel bedeuten können.

N-substituierte Phenothiazinderivate der Formel 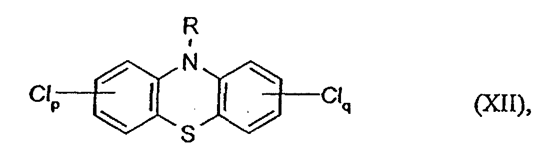 in der
- R: ein Arylrest oder 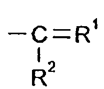 ist, wobei
R¹ Sauerstoff, Schwefel, H₂ oder X₂ bedeutet und
X Br oder Cl ist und
R² einen Arylrest, Br, Cl oder den Rest -CHₓX_{y} bedeutet, in dem
X Br oder Cl und
x einen Wert von 1 bis 3 bedeutet und x + y = 3 ist oder
in der
R ein CF₃-(CF₂)ₙ-CO-Rest ist, bei dem
n für Null, 1, 2 oder 3 steht oder in der
- R: ein -CO-O-Aryl, mit Aryl gleich Phenyl oder gegebenenfalls substituiertem Phenyl steht
- und p + q: stets eine ganz Zahl von Null bis 4 ergibt.

Als Co-Katalysatoren für das erfindungsgemäße Verfahren können weiterhin Heterocyclen die nur Schwefel als Heteroatome oder die gleichzeitig O- und S-Atome enthalten und z.B. den Klassen der Thianthrene oder Phenoxathiine angehören, eingesetzt werden. Das sind z.B. Verbindungen der Formel 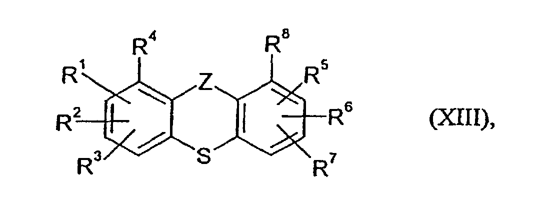 in der Z für -O- oder -S- steht
und
- R¹ bis R⁸: unabhänging voneinander Wasserstoff, Fluor, Chlor, Brom, Nitro, C₁-C₄-Alkyl, C₁-C₄-Acyl, C₁-C₄-Alkoxy, C₁-C₄-Acyloxy, Aryl oder Cyano bedeuten.

In den Formel (I) bis (XIII) stehen jeweils, soweit dort nichts anderes angegeben ist,
Alkyl, auch in zusammengesetzten Gruppierungen wie Alkoxy, Alkylthio, Alkylamino, Alkylsulfonyl, Alkylhydrazino, Carbalkoxy, Dithiocarbalkoxy und Alkoxycarbonyl beispielsweise für C₁-C₈-Alkyl, insbesondere für C₁-C₄-Alkyl,
Aryl, auch in zusammengesetzten Gruppierungen wie Aryloxy, Arylthio und Arylamino beispielsweise für C₆-C₁₀-Aryl, insbesondere für Phenyl,
Acyl, auch in zusammengesetzten Gruppierungen wie Acyloxy, Acylthio, Thioacyl und Acylamino beispielsweise für C₁-C₆-Acyl, insbesondere für C₁-C₃-Acyl,
Halogen, auch in zusammengesetzten Gruppierungen wie Halogencarbonyl, beispielsweise für Fluor, Chlor oder Brom, insbesondere für Fluor oder Chlor, und
Hetaryl bzw. heterocyclische Ringe, auch in zusammengesetzten Gruppierungen wie Hetaryloxy und Hetarylthio beispielsweise für aromatische Ringe, die im Ring 1 bis 3 Heteroatome, ausgewählt aus der Gruppe, die Sauerstoff-, Schwefel- und Stickstoffatome umfaßt, und insgesamt 6 bis 10 Ringatome enthalten.

Bevorzugt eingesetzt werden 1,4-Thiazepinderivate, N-acylierte Phenothiazine, halogen- oder alkylsubstituierte Thianthrene oder Phenoxathine sowie Schwefel oder Schwefelhalogenide. Es ist weiterhin möglich, die Co-Katalysatoren in Kombination mit anderen, nicht als Co-Katalysatoren beschriebenen Elementen oder Verbindungen im erfindungsgemäßen Verfahren einzusetzen. Die Co-Katalysatoren können sowohl einzeln als auch im Gemisch mehrerer von ihnen eingesetzt werden. Die Mengen, in denen man Co-Katalysatoren einsetzen kann, können in weiten Grenzen variieren. Mengen unter 0,001 Gew.-% sind weniger vorteilhaft, da dann die co-katalytische Wirkung nachläßt. Es können sogar Mengen von 5 Gew.-% oder mehr an Co-Katalysator eingesetzt werden, jedoch bieten diese hohen Mengen im allgemeinen keinen Vorteil, verursachen aber gegebenenfalls Nachteile bei der Aufarbeitung. Die erfindungsgemäß zu verwendenden Co-Katalysatoren können daher beispielsweise in einer Menge von 0,001 - 1,0 Gew.-%, bevorzugt 0,001 - 0,5 Gew.-%, besonders bevorzugt 0,005 - 0,1 Gew.-%, jeweils bezogen auf das eingesetzte 3,5-Dimethylfluorbenzol, eingesetzt werden.

Das Molverhältnis von Friedel-Crafts-Katalysator(en) bzw. Vorläufern davon und Co-Katalysator(en) kann im erfindungsgemäßen Verfahren in weiten Grenzen variiert werden. Geeignet ist z.B. ein molares Verhältnis von Friedel-Crafts-Katalysator bzw. Vorläufern davon zu Co-Katalysator vom 100 : 1 bis 1 :100, bevorzugt 75 : 1 bis 1 : 50 und besonders bevorzugt 50 : 1 bis 1 : 10.

Das erfindungsgemäße Verfahren wird zweckmäßigerweise in flüssiger Phase durchgeführt. Gegebenenfalls kann in Verdünnung mit inerten Lösungsmitteln gearbeitet werden. Geeignete Lösungsmittel sind solche, die durch Chlor unter den Bedingungen einer Kernchlorierung nicht angegriffen werden und dem Fachmann hierfür bekannt sind wie Methylenchlorid, Chloroform, Tetrachlorkohlenstoff und Essigsäure. Bevorzugt wird ohne Lösungsmittel gearbeitet.

Als Chlorierungsmittel für das erfindungsgemäße Verfahren können z.B. Chlor oder unter Reaktionsbedingungen Chlor abgebende Substanzen wie Sulfurylchlorid verwendet werden. Chlor ist bevorzugt und kann flüssig oder gasförmig in das Reaktionsgemisch eingeleitet werden. Bevorzugt wird gasförmiges Chlor eingesetzt.

Die Menge des Chlorierungsmittels wird bevorzugt so gewählt, daß ein Chlorierungsgrad nicht wesentlich höher als 2 resultiert. Höhere Chlorierungsgrade sind möglich, aber nicht vorteilhaft, da sie zur Bildung von 2,4,6-Trichlor-3,5-dimethylfluorbenzol führen. Andererseits kann es vorteilhaft sein, deutlich weniger als 2 mol Chlorierungsmittel pro mol 3,5-Dimethylfluorbenzol einzudosieren, um die Bildung des Trichlorproduktes nahezu völlig zu vermeiden. Das Chlorierungsmittel wird daher beispielsweise in einer Menge von 1,0 bis 2,2 mol, bevorzugt von 1,25 bis 2,1 mol, besonders bevorzugt von 1,5 bis 2,0 mol pro mol 3,5-Dimethylfluorbenzol eingesetzt.

Die erfindungsgemäß durchzuführende Kernchlorierung kann grundsätzlich bei einer Temperatur vom Erstarrungspunkt bis zum Siedepunkt des Reaktionsgemisches durchgeführt werden. Im allgemeinen liegt die Reaktionstemperatur bei 0 bis 120°C, bevorzugt bei 20 bis 100°C, besonders bevorzugt bei 30 bis 90°C. Die Temperatur wird in den angegebenen Grenzen im Verlaufe des Verfahrens so gewählt, daß das Chloriergemisch immer flüssig oder nahezu flüssig bleibt.

Der Reaktionsdruck kann normal, vermindert oder erhöht sein und ist grundsätzlich unkritisch. Wegen der kostengünstigen Durchführung ist Normaldruck bevorzugt. Erhöhter Druck kann beispielsweise dann angezeigt sein, wenn oberhalb des Siedepunktes eines tiefsiedenden Lösungsmittels gearbeitet werden soll. In diesem Fall kann beispielsweise unter dem sich von selbst einstellenden Eigendruck des Reaktionsgemisches gearbeitet werden.

Der Wassergehalt der Reaktionsmischung ist im allgemeinen unkritisch. Es ist bevorzugt, alle Einsatzstoffe nicht speziell zu trocknen, sondern sie mit dem (geringen) Wassergehalt einzusetzen, mit dem sie üblicherweise in der chemischen Technik vorliegen. Es ist jedoch auch möglich, einzelne oder alle Stoffe des Reaktionsgemisches speziell zu trocknen. Üblicherweise sollte der Wassergehalt der Einsatzstoffe nicht über den Sättigungsgrenzen der jeweiligen Einsatzstoffe liegen. Erfindungsgemäß bevorzugt sind Wassergehalte im Chloriergemisch von weniger als 250 ppm, besonders bevorzugt weniger als 150 ppm, ganz besonders bevorzugt weniger als 100 ppm.

Für die praktische Durchführung des erfindungsgemäßen Verfahrens ist die Reihenfolge der Zugabe der einzelnen Komponenten zum Reaktionsgemisch beliebig. Das Verfahren kann man sowohl kontinuierlich als auch diskontinuierlich durchführen. Beispielsweise legt man 3,5-Dimethylfluorbenzol bei der gewünschten Reaktionstemperatur vor, gibt Friedel-Crafts- und schwefelhaltigen Co-Katalysator zu und dosiert das Chlorierungsmittel bis zum gewünschten Chloriergrad ein.

Falls die Chlorierung ohne Lösungsmittel durchgeführt wird, können bei relativ niedrigen Reaktionstemperaturen. z.B. bei solchen unter 45°C, ab der Zugabe von z.B. 1,6 mol Chlorierungsmittel pro 3,5-Dimethylfluorbenzol feste Anteile aus dem Chloriergemisch ausfallen. Wenn man in solchen Fällen noch weiter chlorieren möchte, so kann man das Gemisch durch Temperaturerhöhung auf z.B. mindestens 75°C nahezu vollständig flüssig halten.

Anschließend wird das Reaktionsgemisch aufgearbeitet, z.B. durch Destillation. Das im Chloriergemisch mit enthaltene 4-Chlor-3,5-dimethylfluorbenzol kann durch Destillation abgetrennt und zur nächsten erfindungsgemäßen Chlorierung zurückgeführt werden. Danach kann die Dichlor-3,5-dimethylfluorbenzol-Fraktion abgetrennt werden. Die Isomeren-Selektivität ist beim erfindungsgenäßen Verfahren so hoch, daß diese Fraktion i.a. deutlich über 98 Gew.-% des gewünschten 2,4-Dichlor-3,5-dimethyl-fluorbenzol enthält.

Die folgenden Beispiele sollen das erfindungsgemäße Verfahren verdeutlichen, ohne es jedoch einzuschränken.

### Beispiele

### Beispiel 1

In einem Chlorierbecher wurden 124,0 g 3,5-Dimethylfluorbenzol, 0,12 g FeCl₃ und 0,05 g N-Trifluoracetylphenothiazin bei 40°C vorgelegt und mit 107 g Chlor in 6 Stunden gleichmäßig schnell begast. Das erhaltene Produktgemisch wurde mittels GC analysiert. Es enthielt 44,80 % 4-Chlor-3,5-dimethylfluorbenzol, 53,76 % 2,4-Dichlor-3,5-dimethylfluorbenzol, 0,95 % 2,6-Dichlor-3,5-dimethylfluorbenzol und 0,14 % 2,4,6-Trichlor-3,5-dimethylfluorbenzol (Rest: unbekannte Produkte). Die Bildungsselektivität für die Summe aus dem 4-Chlor-3,5-dimethylfluorbenzol und 2,4-Dichlor-3,5-dimethylfluorbenzol betrug somit 98,56 %.

### Beispiel 2

In einem Chlorierbecher wurden 124,0 g 3,5-Dimethylfluorbenzol, 0,25 g FeCl₃ und 0,054 g Co-Katalysator der Formel 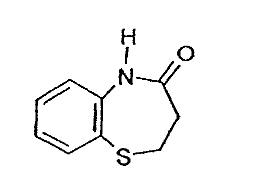 vorgelegt. Bei 40°C wurden 113 g Chlor, dann bei 40 bis 77°C ansteigend weitere 30 g Chlor eingeleitet (Gesamtchlorierzeit 5 Stunden). Das erhaltene Produkt wurde mit GC analysiert. Es enthielt 1,21 % 4-Chlor-3,5-dimethylfluorbenzol, 94,47 % 2,4-Dichlor-3,5-dimethylfluorbenzol, 0,24 % 2,6-Dichlor-3,5-dimethylfluorbenzol und 3,85 % 2,4,6-Trichlor-3,5-dimethylfluorbenzol (Rest: unbekannte Produkte). Die berechnete Isomereneinheit der Dichlorfraktion betrug somit 99,75 %.

### Beispiel 3

Das Verfahren von Beispiel 2 wurde wiederholt, jedoch wurden als Co-Katalysator 0,059 g der Verbindung der Formel 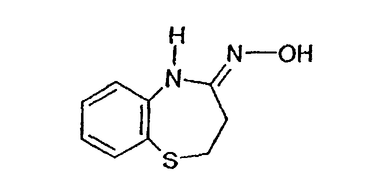 eingesetzt. Bei 40°C wurden 110 g Chlor, dann bei 40 bis 60°C weitere 15 g Chlor eingeleitet (Gesamtchlorierzeit 5 Stunden). Die GC-Analyse des Chloriergemisches ergab folgende Zusammensetzung: 18,98 % 4-Chlor-3,5-dimethylfluorbenzol, 79,99 % 2,4-Dichlor-3,5-dimethylfluorbenzol, 0,76 % 2,6-Dichlor-3,5-dimethylfluorbenzol und 0,17 % 2,4,6-Trichlor-3,5-dimethylfluorbenzol (Rest: unbekannte Produkte).

### Beispiel 4

In einem Chlorierbecher wurden 124,0 g 3,5-Dimethylfluorbenzol, 0,24 g FeCl₃ und 0,82 g Schwefel vorgelegt. Bei 40°C wurden 119 g Chlor, dann bei 40-58°C weitere 5 g Chlor eingeleitet (Chlorierzeit 5 Stunden). Die GC-Analyse ergab folgende Zusammensetzung: 24,67 % 4-Chlor-3,5-dimethylfluorbenzol, 73,34 % 2,4-Dichlor-3,5-dimethylfluorbenzol, 0,38 % 2,6-Dichlor-3,5-dimethylfluorbenzol und 0,52 % 2,4,6-Trichlor-3,5-dimethylfluorbenzol (Rest: unbekannte Produkte). Die Bildungsselektivität für die Summe aus dem 4-Chlor-3,5-dimethylfluorbenzol und 2,4-Dichlor-3,5-dimethylfluorbenzol betrug somit 98,01 %.

### Beispiel 5

Das Verfahren des Beispiels 4 wurde wiederholt, jedoch wurden nur 0,12 g FeCl₃ und statt Schwefel 0,09 g Dischwefelchlorid eingesetzt. 106 g Chlor wurden bei 40°C und weitere 36 g Chlor bei 40 bis 79°C eingeleitete (Chlorierzeit 5 Stunden). Die GC-Analyse ergab folgende Zusammensetzung des Chloriergemisches: 2,10 % 4-Chlor-3,5-dimethylfluorbenzol, 93,81 % 2,4-Dichlor-3,5-dimethylfluorbenzol, 0,17 % 2,6-Dichlor-3,5-dimethylfluorbenzol und 2,56 % 2,4,6-Trichlor-3,5-dimethylfluorbenzol (Rest: unbekannte Produkte). Die berechnete Isomereneinheit der Dichlorfraktion betrug somit 99,8 %.

### Beispiel 6

In einem Chlorierbecher wurden 124,0 g 3,5-Dimethylfluorbenzol, 0,3 g FeCl₃ und 0,4 g Co-Katalysator der Formel 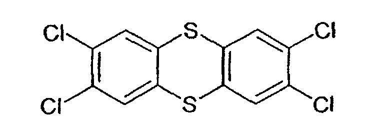 bei 60°C vorgelegt. bei dieser Temperatur wurden 124 g Chlor im Verlaufe von 8 Stunden gleichmäßig schnell eingeleitet. Die GC-Analyse des Chloriergemisches ergab: 21,09% 4-Chlor-3,5-dimethylfluorbenzol, 77,23% 2,4-Dichlor-3,5-dimethylfluorbenzol, 1,13 % 2,6-Dichlor-3,5-dimethylfluorbenzol und 0,31 % 2,4,6-Trichlor-3,5-dimethylfluorbenzol (Rest: unbekannte Produkte).

### Beispiel 7

Das Verfahren des Beispiels 6 wurde wiederholt, jedoch wurden als Co-Katalysatoren 0,4 g Verbindungen der Formel 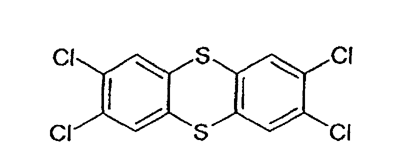 in Form eines Isomerengemisches eingesetzt, das durch Chlorierung von 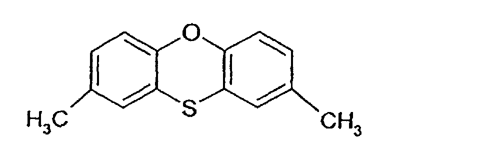 mit 4 Mol Cl₂ erhalten worden war (siehe EP-A 173 222).

Die GC-Analyse des Chloriergemisches ergab folgende Zusammensetzung: 20,55 % 4-Chlor-3,5-dimethylfluorbenzol, 77,50 % 2,4-Dichlor-3,5-dimethylfluorbenzol, 1,07 % 2,6-Dichlor-3,5-dimethylfluorbenzol und 0,48 % 2,4,6-Trichlor-3,5-dimethylfluorbenzol (Rest: unbekannte Produkte).

## Patentansprüche

1. Verfahren zur Herstellung von 2,4-Dichlor-3,5-dimethylfluorbenzol, **dadurch gekennzeichnet, daß** man 3,5-Dimethylfluorbenzol in Gegenwart von Friedel-Crafts-Katalysatoren und einem schwefelhaltigen Co-Katalysator chloriert.

2. Verfahren nach Anspruch 1, **dadurch gekennzeichnet, daß** man als Friedel-Crafts-Katalysatoren Antimonchloride, Antimonoxychloride, Aluminiumchlorid, Eisen(II)-chlorid, Eisen(III)chlorid, Tellurchloride, Bleichloride, Molybdänchloride, Zinnchloride, Wolframchloride, Titanchloride, Zinkchloride, Bortrichlorid und/oder Bortrifluorid oder Elemente und Elementverbindungen einsetzt, die während der Chlorierung einen Friedel-Crafis-Katalysator bilden.

3. Verfahren nach Ansprüchen 1 und 2, **dadurch gekennzeichnet, daß** man 0,0005 bis 5 Gew.-% Friedel-Crafts-Katalysator bzw. eines Vorläufers dafür einsetzt, bezogen auf die Menge des eingesetzten 3,5-Dimethylfluorbenzols.

4. Verfahren nach Ansprüchen 1 bis 3, **dadurch gekennzeichnet, daß** als schwefelhaltige Co-Katalysatoren Schwefel, Schwefelhalognide oder Heterocyclen einsetzt, die gleichzeitig N- und S-Atome enthalten.

5. Verfahren nach Ansprüchen 1 bis 4, **dadurch gekennzeichnet, daß** man als Co-Katalysatoren Heterocyclen aus den Klassen Thiazine, Thiazepine, Thiazocine, Phenoxathiine und Thianthrene einsetzt.

6. Verfahren nach Ansprüchen 1 bis 5, **dadurch gekennzeichnet, daß** man Co-Katalysatoren in Mengen von 0,001 bis 5 Gew.-%, bezogen auf das eingesetzte 3,5-Dimethylfluorbenzol, einsetzt.

7. Verfahren nach Ansprüchen 1 bis 6, **dadurch gekennzeichnet, daß** das Molverhältnis von Friedel-Crafts-Katalysator bzw. Vorläufern davon zu Co-Katalysator 100:1 bis 1:100 beträgt.

8. Verfahren nach Ansprüchen I bis 7, **dadurch gekennzeichnet, daß** man als Chlorierungsmittel Chlor oder eine unter Reaktionsbedingungen Chlor abgebende Substanz in einer Menge von 1,0 bis 2,2 Mol pro Mol 3,5-Dimethylfluorbenzol einsetzt.

9. Verfahren nach Ansprüchen 1 bis 8, **dadurch gekennzeichnet, daß** man es bei Reaktionstemperaturen im Bereich 0 bis 120°C und Normaldruck durchführt, wobei man die Temperatur jedoch so wählt, daß das Chloriergemisch flüssig oder nahezu flüssig bleibt.

## Claims

1. Process for the preparation of 2,4-dichloro-3,5-dimethylfluorobenzene, **characterized in that** 3,5-dimethylfluorobenzene is chlorinated in the presence of Friedel-Crafts catalysts and a sulphur-containing co-catalyst.

2. Process according to Claim 1, **characterized in that** the Friedel-Crafts catalysts used are antimony chlorides, antimony oxychlorides, aluminium chloride, iron(II)chloride, iron(III)chloride, tellurium chlorides, lead chlorides, molybdenum chlorides, tin chlorides, tungsten chlorides, titanium chlorides, zinc chlorides, boron trichloride and/or boron trifluoride or elements and compounds of elements which, during chlorination, form a Friedel-Crafts catalyst.

3. Process according to Claims 1 and 2, **characterized in that** 0.0005 to 5% by weight of Friedel-Crafts catalyst or of a precursor thereof are employed, based on the amount of 3,5-dimethylfluorobenzene employed.

4. Process according to Claims 1 to 3, **characterized in that** the sulphur-containing co-catalysts employed are sulphur, sulphur halides or heterocycles which simultaneously comprise N and S atoms.

5. Process according to Claims 1 to 4, **characterized in that** the co-catalysts employed are heterocycles of the classes of the thiazines, thiazepines, thiazocines, phenoxathiines and thianthrenes.

6. Process according to Claims 1 to 5, **characterized in that** the co-catalysts are employed in amounts of 0.001 to 5% by weight, based on 3,5-dimethylfluorobenzene employed.

7. Process according to Claims 1 to 6, **characterized in that** the molar ratio of Friedel-Crafts catalyst or precursors thereof to co-catalyst is 100 : 1 to 1 : 100.

8. Process according to Claims 1 to 7, **characterized in that** the chlorinating agent employed is chlorine or a substance which liberates chlorine under the reaction conditions, in an amount of 1.0 to 2.2 moles per mole of 3,5-dimethylfluorobenzene.

9. Process according to Claims 1 to 8, **characterized in that** the reaction temperatures range from 0 to 120°C and the pressure is atmospheric, the temperature, however, being chosen in such a way that the chlorination mixture remains liquid or virtually liquid.

## Revendications

1. Procédé pour la préparation du 2,4-dichloro-3,5-diméthylfluorobenzène, **caractérisé en ce qu'**on soumet à une chloration du 3,5-diméthylfluorobenzène en présence de catalyseurs de Friedel-Crafts et d'un cocatalyseur contenant du soufre.

2. Procédé selon la revendication 1, **caractérisé en ce qu'**on met en oeuvre, à titre de catalyseurs de Friedel-Crafts, des chlorures d'antimoine, des oxychlorures d'antimoine, du chlorure d'aluminium, du chlorure de fer(II), du chlorure de fer(III), des chlorures de tellure, des chlorures de plomb, des chlorures de molybdène, des chlorures d'étain, des chlorures de tungstène, des chlorures de titane, des chlorures de zinc, le trichlorure de bore et/ou le trifluorure de bore ou encore des éléments et des composés élémentaires qui forment un catalyseur de Friedel-Crafts au cours de la chloration.

3. Procédé selon les revendications 1 et 2, **caractérisé en ce qu'**on met en oeuvre, un catalyseur de Friedel-Crafts respectivement un précurseur de ce dernier, à concurrence de 0,0005 à 5 % en poids rapportés à la quantité du 3,5-diméthylfluorobenzène mis en oeuvre.

4. Procédé selon les revendications 1 à 3, **caractérisé en ce qu'**on met en oeuvre, à titre de cocatalyseurs contenant du soufre, du soufre, des halogénures de soufre ou encore des composés hétérocycliques qui contiennent simultanément des atomes d'azote et des atomes de soufre.

5. Procédé selon les revendications 1 à 4, **caractérisé en ce qu'**on met en oeuvre à titre de cocatalyseurs, des composés hétérocycliques choisis parmi les classes des thiazines, des thiazépines, des thiazocines, des phénoxathiines et des thianthrènes.

6. Procédé selon les revendications 1 à 5, **caractérisé en ce qu'**on met en oeuvre des cocatalyseurs dans des quantités de 0,001 à 5 % en poids rapportés au 3,5-diméthyl-fluorobenzène mis en oeuvre.

7. Procédé selon les revendications 1 à 6, **caractérisé en ce que** le rapport molaire du catalyseur de Friedel-Crafts respectivement des précurseurs de ce dernier au cocatalyseur s'élève de 100 : 1 à 1 : 100.

8. Procédé selon les revendications 1 à 7, **caractérisé en ce qu'**on met en oeuvre, à titre d'agent de chloration, du chlore ou une substance cédant du chlore dans les conditions réactionnelles, en une quantité de 1,0 à 2,2 moles par mole du 3,5-diméthylfluorobenzène.

9. Procédé selon la revendication 1 à 8, **caractérisé en ce qu'**on le met en oeuvre à des températures réactionnelles dans la plage de 0 à 120 °C et sous pression normale, en sélectionnant néanmoins une température telle que le mélange de chloration reste en phase liquide ou pratiquement liquide.
